# EUROPEAN PATENT SPECIFICATION

(11) **EP 0 975 360 B1**
(45) Date of publication and mention of the grant of the patent: **02.08.2006**
(21) Application number: 98907727.6
(22) Date of filing: 04.03.1998
(51) Int. Cl.: A61K 39/00

(54) **BOVINE RESPIRATORY AND ENTERIC CORONOVIRUS AS A VACCINE**
BOVINER ATMUNGS- UND DARMCORONAVIRUS ALS IMPFSTOFF
CORONAVIRUS RESPIRATOIRE BOVIN UTILISE COMME VACCIN

(30) Priority: 10.03.1997 US 814311
(43) Date of publication of application: 02.02.2000
(73) Proprietor: BAYER CORPORATION, Pittsburgh, PA 15205-9741 (US); Immtech Biologics, LLC., Bucyrus, KS 66013 (US)
(72) Inventor: STINE, Lisa, C., Olathe, KS 66062 (US); McGINLEY, Michael, J., Des Moines, IA 50317 (US); ANDERSON, Gary, A., Bucyrus, KS 66013 (US); STINE, Douglas, L., Olathe, KS 66062 (US); BROWN, Karen, K., Parkville, MO 64152 (US); DORY, Andre, D., Gladstone, MO 64119 (US); LIEM, Adrian, Lenexa, KS 66220 (US)
(74) Representative: Schumacher, Günter
(86) International application number: PCT/US1998/004392
(87) International publication number: WO 1998/040097

(56) References cited:
- WO-A-95/34686
- WO-A-96/41874
- J. STORZ ET AL.: "Coronavirus isolation from nasal swap samples in cattle with signs of respiratory-tract disease after shipping." JOURNAL OF THE AMERICAN VETERINARY MEDICAL ASSOCIATION, vol. 208, no. 9, 1 May 1996, pages 1452-1455, XP002084556 Chicago, IL, USA cited in the application
- K. MÖSTL ET AL.: "Incidence of diarrhoea and of rotavirus- and coronavirus-shedding in calves, whose dams had been vaccinated with an experimental oil-adjuvanted vaccine containing bovine rotavirus and bovine coronavirus." JOURNAL OF VETERINARY MEDICINE, vol. 35, no. 3, April 1988, pages 186-196, XP002084557 Hamburg, Germany cited in the application
- E. THURBER ET AL.: "Field trial evaluation of a reo-coronavirus calf diarrhea vaccine." CANADIAN JOURNAL OF COMPARATIVE MEDICINE, vol. 41, no. 2, April 1977, pages 131-136, XP002084558 Ottawa, Canada cited in the application
- L. MYERS ET AL.: "Colostral and milk antibody titers in cows vaccinated with a modified live-rotavirus-coronavirus vaccine." JOURNAL OF THE AMERICAN VETERINARY MEDICAL ASSOCIATION, vol. 181, no. 5, 1 September 1982, pages 486-488, XP002084559 Chicago, IL, USA cited in the application
- C. MEBUS ET AL.: "Neonatal calf diarrhea: propagation, attenuation, and characteristics of a coronavirus-like agent." AMERICAN JOURNAL OF VETERINARY RESEARCH, vol. 34, no. 2, 1973, pages 145-150, XP002084560 Schaumburg, USA cited in the application

## Description

Field of the Invention: The present invention relates to a bovine respiratory coronavirus that can be used in a modified live form, an inactivated form or a subunit form to produce a vaccine which protects from diseases caused by bovine respiratory coronavirus (BRCV) and bovine enteric coronavirus (BECV). The invention also relates to a bovine enteric coronavirus which can be used in a modified live form, an inactivated form or a subunit form to produce a vaccine which protects from diseases caused by either bovine respiratory coronavirus or bovine enteric coronavirus and methods for making and using said vaccines.

Brief Description of the Prior Art: Respiratory disease breaks in vaccinated herds have raised questions about other viruses or bacteria being involved in causing Bovine respiratory disease complex (shipping fever). The primary causes of the disease complex have been identified as four viruses: infectious bovine rhinotracheitis virus (IBRV), parainfluenza virus type 3 (PI₃), bovine virus diarrhea virus (BVDV) and bovine respiratory syncytial virus (BRSV). To protect against the disease complex, the art has used vaccination programs that include use of vaccines for all of these viruses either in a singular form (monovalent) or in a combination form (bivalent if any of two of the viruses are present in a vaccine or multivalent if more than two viruses are present in a vaccine). In the past, these vaccines seemed to be effective. However, control of bovine respiratory disease complex with these vaccines has recently been questioned because of respiratory disease breaks in vaccinated herds, indicating the possibility of other viruses or bacteria being involved.

A bovine coronavirus was isolated from the respiratory tract of calves having respiratory symptoms as early as 1984 by McNulty et al (Vet. Micro., 1984, 9: 425 - 434). Since that time there has been a significant controversy about whether coronaviruses isolated from the respiratory tract of bovines (BRCV) is the causative agent for the disease breaks in herds of cattle vaccinated with current bovine respiratory vaccines. If so, there is also a controversy about whether BRCV is the same as coronaviruses isolated from the enteric tract of bovines (BECV).

BECV, first isolated in the 1970s by Mebus et al . (Am J Vet Res, 1973, 34:145-150), is now widely recognized as an important cause of neonatal calf diarrhea. It has also been recognized as associated with winter diarrhea in adult cattle. In the propagation of BECV, the art has employed numerous cell types including primary cells (tracheal organ and gut culture) and several cell lines. Examples of the cell lines include: human rectal tumor cells (ATCC designation HRT-18), Vero cells, Madin Darby Bovine kidney (MDBK) cells and Madin Darby canine kidney 1 (MDCK1) cells. Addition of exogenous trypsin enhances or promotes the growth of BECV in these and in many other cell lines. BECV propagation in early passages in these cell cultures is typically without the production of a recognizable cytopathic effect (CPE). Later passages result in a marked CPE characterized by syncytia formation and cell detachment.

A vaccine has been produced and is available with a claim for protection of calves against enteric disease caused by BECV. It is administered to dams prior to calving. The vaccine's protective capability has been questioned because it relies on passive immunization (Myers and Snodgrass, 1982, J Am Vet Med Assoc 181: 486-488 and Mostl and Burki, 1988, J Vet Med : 35, 186-196). Immunization of calves by administering modified live BECV by the oral route has protected colostrum deprived calves in experimental trials but has not proved effective in field trials (Thurber et al., 1977, Can. J. Comp. Med., 41: 131-136).

As would be realized from the foregoing, the increased incidence respiratory disease in feedlots, winter dysentery of adult dairy and beef cattle and neonatal calf diarrhea, potentially caused by BRCV and BECV indicates that a solution to the disease problem is needed. There is a debate in the scientific community about whether BRCV and BECV are variants of the same virus. Dr. Johannes Storz, the recognized leader in this area has recently published that there are significant *in vitro* and *in vivo* differences between BRCV and BECV and that because of these differences they are distinctly different viruses and would not be expected to cross-protect (Storz, 1996, JAVMA, 208:9, 1452-1455). These significant differences between BRCV and BECV are: 1) BRCV can be readily isolated from field samples on first passage, without the aid of trypsin, using a cloned human rectal tumor (cHRT) cell line and BRCV isolates propagated in this manner show a distinct and enhanced cell fusing cytopathology. By contrast, wild type BECV isolations cannot typically be made without multiple passages or trypsin enhancement or both and do not exhibit enhanced cell fusing cytopathology; 2) BRCV and BECV isolates have distinct *in vitro* hemagglutination patterns wherein BECV hemagglutinates rodent and chicken red blood cells (RBCs) while BRCV isolates only hemagglutinate rodent RBCs; 3) BECV is a major cause of viral diarrhea in the young calf while BRCV has been isolated from a high percentage of cattle presenting with respiratory symptoms such as coughing, dyspnea, nasal discharge, and elevated body temperature. Because of such differences, Storz appears to teach away from the use of a BRCV vaccine to protect against disease caused by both BRCV and BECV or a BECV vaccine protecting against disease caused by either BECV or BRCV.

### SUMMARY OF THE INVENTION

The present invention also encompasses an improved cHRT cell line and the processes for using the same for propagation of BRCV or BECV to a high titer so as to produce a high antigenic mass that is useful in preparing a commercially feasible vaccine. By the term "high antigenic mass" is meant an immunogenically effective amount of a virus or an antigen derived from the virus which is useful in immunizing bovines to provide protection against disease caused by BRCV or BECV. By the term "commercially feasible" is meant that the vaccine can be cost effectively produced. For example, a commercially feasible vaccine would not require cost prohibitive levels of concentration of the antigenic mass in order to reach a effective antigenic mass for vaccines. One such cHRT cell line, HRT-18G, has been deposited with the American Type Culture Collection by Dr. Storz and was assigned Accession No CRL11663. A second, more effective cHRT cell line, has been developed by the inventors and designated HRT-E6. This cell line was deposited with the American Type Culture Collection (ATCC), 12301 Parklawn Drive, Rockville, Md 20852 on April 6, 1998 under the Accession Number CRL 12478.

The present invention further encompasses a method for isolating the improved (cloned) cHRT cell line designated as HRT-E6. The cloning process selectively provided a cHRT cell which propagates BRCV to a higher titer than that obtained with the parent cell with ATCC designation CCL 244 or with the art-related HRT-18G cells.

The present invention additionally encompasses a method for propagating the BRCV or BECV viruses to a high titer, producing a high antigenic mass, in a cHRT cell line such as the HRT-E6 such that a commercially feasible vaccine can be produced.

It has been found that both an inactivated, adjuvanted, and a modified live BRCV, vaccine stimulated a neutralizing antibody response in previously-seronegative calves in a significant amount to provide adequate immunological activity indicative of protection and an immunogenically effective amount. It is also envisioned that immunization of calves with coronavirus would reduce respiratory and enteric coronavirus disease following an intranasal challenge with a virulent BRCV.

### DETAILED DESCRIPTION OF THE INVENTION

As set forth above, the present invention is directed to a modified live, an inactivated or a subunit form of a vaccine or a combination thereof for protection of bovines from diseases caused by BRCV or BECV, comprising an immunogenically effective amount of a BRCV or BECV isolate(s) or antigens therefrom. The BRCV or BECV isolate(s) can be obtained by inoculating susceptible cell cultures with samples from a diseased animal such as a calf. A subunit can be obtained by extraction from the virus or from an expression as a recombinant by a non-BRCV or non-BECV organism.

Another embodiment of the invention includes immunization of bovines to protect against disease caused by either BRCV or BECV, comprising an immunogenically effective amount of BECV or BRCV isolate(s) or antigens therefrom. The BECV or BRCV isolate(s) or its antigens can be in the form of a modified live virus, an inactivated virus or a subunit wherein the subunit is obtained by extraction from the virus or from an expression as a recombinant by a non-BECV or non-BRCV organism. It is within the scope of the invention that any of the above may be combined and that an adjuvant and a pharmaceutically acceptable carrier may be added. The virus is propagated in a high susceptibility cell line such as a cHRT cell line.

A further embodiment of the invention comprises an improved cloned human rectal tumor cell, HRT-E6, designated as ATCC CRL RL 12478 The improvement comprises a substantially more effective propagation of BRCV and BECV viruses on these cells lines than either its parent cell, CCL 244, or another cHRT cell, HRT-18G. This improved cHRT cell designated herein as HRT-E6 can be produced by using art-known limiting-dilution techniques in 96-well tissue culture plates. These techniques involve growing CCL 244 cells in a vessel such as a roller bottle, removing the cells by art-known trypsin treatment (using a TrypsinEDTA solution containing from 0.05% to 0.25% trypsin combined with 0.04% EDTA), counting the viable cells removed from the vessel and making 10-fold or other equally useful dilutions of the cells in Dulbecco's Minimal Essential Medium (DMEM) or Minimal Essential Medium (MEM) plus serum, preferably fetal bovine serum or horse serum, so that a large portion of the wells in the 96-well plate would theoretically contain only one cell. The cells are microscopically observed to determine which wells truly contain a single cell. Once the wells initially containing a single cell become confluent, the cells in each well are removed by art-known trypsinization techniques and passaged until a large volume of cells are obtained. Each large volume of cells originating from a single cell is defined as a clone and is given a separate number. Each clone is tested for high susceptibility to BRCV and BECV by inoculation with BRCV or BECV isolates and selecting those which demonstrate high titers. Illustratively, a susceptible cell would propagate BRCV or BECV in amounts sufficient to produce a cytopathic effect (CPE). The more susceptible clones would propagate BRCV or BECV to titers of 10 ^{3.0} tissue culture infective dose (TCID₅₀/mL) The most susceptible clones, categorized herein as high-susceptibility cells, propagate BRCV and BECV in amounts of 10 ^{5.0} TCID₅₀/mL. As such, the most susceptible clones are sequentially passaged to eventually produce a high susceptibility cell line which may be readily used for propagation of BRCV or BECV to provide a commercially-feasible vaccine, whereas the more susceptible clones may be readily used for propagation and isolation of BRCV or BECV to diagnose disease.

A still further embodiment of the invention is a method of propagating BRCV or BECV to a high antigenic mass yielding an immunologically effective amount of BRCV, BECV or antigens therefrom. The method comprises the steps of: (1) propagating a high-susceptibility cell on a vessel surface or in suspension in the presence of a tissue culture medium to effectively produce the cells at an acceptable viable count which is at least 1 X 10⁵/mL; (2) inoculating the high-susceptibility cell with BRCV or BECV to produce an infected cell culture; (3) incubating the infected cell culture at 30 to 38°C until acceptable CPE is produced, preferably at between 35 and 37°C; (4) harvesting the resulting infected cell culture by transfer into a collecting container; (5) optionally disrupting the remaining whole cells in the harvested infected cell culture by methods including, but not limited to, microfluidization, freeze-thaw and sonication; and (6) optionally concentrating the infected cell culture to a high antigenic mass. The concentration step can be carried out by art-known means including but not limited to ultrafiltration, centrifugation, settling or chromatography. Although any high-susceptibility cell can be used to propagate the BRCV and BECV, the use of cHRT cells is preferable with HRT-E6, designated as ATCC CRL CRL 12478 being more preferable. The following is a more specific but non-limiting description of a method of propagation. The cHRT cells such as HRT-E6, are grown in a vessel such as a roller bottle or on microcarriers in a bioreactor in a tissue culture medium such as Dulbecco's Minimal Essential medium supplemented with a serum such as 1-10% bovine serum or donor horse serum and a suitable buffer system such as 1.3g/L sodium bicarbonate. Cell passages are made with cell counts sufficient to attain desired confluency or cell density of the cells on the surface of the vessel or microcarriers or in suspension, within 24-48 hours. Confluent monolayers, suspended cells or microcarriers have their growth medium removed and then are inoculated with BRCV or BECV to produce a multiplicity of infection (MOI) of between 0.001 and 0.1, preferably between 0.01 and 0.1. The resulting viruses may be first adsorbed onto the monolayers or combined with cells in suspension and then maintenance medium may be added. The maintenance medium is essentially the same as the cell growth medium described above, except that a reduced amount of serum is added. The resulting virus-infected tissue cultures are incubated at between 30 and 38°C, preferably between about 35 and 37°C until complete CPE is observed. Typically, complete CPE is observed between 1 and 7 days post infection, preferably between 2 and 4 days post infection. The virus-infected tissue cultures displaying acceptable CPE are collected (harvested) into a single container producing harvest fluids. An acceptable CPE for purposes of this invention is indicated by at least 50% destruction of the cell sheet.

The harvest fluids may be inactivated with any of several inactivating agents to prepare the inactivated form of the vaccine. The inactivating agents will be selected from the group consisting of beta propiolactone, formaldehyde, binary ethylenimine, heat and UV light exposure.

The harvest may also be used in a live form if the BRCV or BECV has been previously attenuated using art-known techniques. Illustrative but non-limiting examples of attenuation can be multiple passage in tissue culture and treatment with mutagenic agents, in order to select a mutant which is unable to produce disease when injected intranasally into young calves. Additionally, live harvest may be used without production of mutations by administration of the vaccine made therefrom via atypical routes including intramuscular, subcutaneous or intradermal.

In producing a subunit vaccine, BRCV or BECV is grown as described above and the virus harvest is extracted with any of several agents including but not limited to detergents and organic solvents. Extracts may be used in an extracted form or may be further purified by ultrafiltration and/or column chromatography and then combined with an adjuvant for formulation into a vaccine. A specific example of this method comprises infecting cHRT cells with BRCV according to the method described previously. The infected cells are then harvested when CPE is >80%. Harvested infected cells are separated from the fluids by low-speed centrifugation and the collected pellets are extracted by addition of a detergent in a buffer system. The buffer-detergent system consists of phosphate buffered saline (PBS) or any other buffer which is non-toxic for tissue culture cells plus 1.0 to 10.0% detergent such as IGEPAL CA-360, available from Sigma Chemical Company. Preferably, 1.0 to 2.0% detergents selected from the group consisting of IGEPAL CA-360, Triton-X-100 (available from Sigma Chemical Company) and sodium dodecylsulfate (also available from Sigma_Chemical Company) are used Organic solvents are used at the same concentrations and include but are not limited to alcohols, esters or ethers. Any buffer system can be used as long as it is non-toxic for tissue culture cells. Other buffers which are useful include but are not limited to other salts such as sulfates and carbonates and organic buffers such as Tris[hydroxymethyl]aminomethane (TRIS), N,N'-bis[2-ethanesulfonic acid]: 1,4-piperazinediethanesuffonic acid (PIPES), and N-[2-Hydroxyethylpiperazine-N'-]2-ethanesulfonic acid (HEPES), all available from Sigma Chemical Company. This buffer-detergent system is used to resuspend the cell pellet and extraction is carried out by mixing the suspended infected cell pellet at a controlled temperature between 4°C and 37°C, preferably between 4°C and 10°C until the cell pellet is uniformly solubilized (generally between 30 and 120 minutes). Following this extraction, any insoluble material is removed by low speed centrifugation (batch or continuous flow) and may be re-extracted as described above. Solubilized extracts are then combined and purified by column chromatography (size exclusion, lectin or other affinity, anion/cation exchange, and/or reverse phase) before adjuvanting or extracts may be adjuvanted directly. The antigenic mass is measured by art-known methods such as enzyme linked immunoassay (ELISA), HPLC, FPLC or electrophoresis prior to adjuvanting. If the antigenic mass is high enough the extract or purified extract may be diluted in PBS. If the antigenic mass is too low to be immunogenically effective, the extract or purified extract may be concentrated using ultrafiltration, centrifugation or other such concentration methods. Adjuvanting can be conducted using any of the adjuvants described below.

Adjuvants may be used with the inactivated or modified live harvest fluids or with subunit or recombinant antigens derived from BRCV or BECV in preparation of a vaccine. If an adjuvant is used for preparation of a vaccine, it is understood that any adjuvant can be added to increase the effectiveness of the vaccine. Such adjuvants would include but are not limited to polymers or block co-polymers including Carbopol®, DEAE Dextran, Dextran sulfate, methacrylates and POLYGEN^{™}; IMMUGEN^{™}; aluminum salts such as aluminum hydroxide and aluminum phosphate; Avridine; Lipid A, dimethyldodecylammonium bromide; poxvirus proteins such as Baypamune; oils such as SUPRIMM^{™}, EMULSIGEN^{™}, EMULSIGEN PLUS^{™}; animal oils such as squalene; mineral oils such as Drakeol and Montanides; vegetable oils such as peanut oil; triterpenoid glycosides such as saponin, QuilA, and QS21; detergents such as Tween-80 and Pluronics; bacterial component adjuvants such as from Corynebacterium, Propionibacterium, and Mycobacterium; interleukins, monokines, and interferons; liposomes; ISCOMs; synthetic glycopeptides such as muramyl dipeptides and derivatives thereof; cholera toxin; or combinations of the above.

This invention describes a method for propagating a BRCV by growing the virus in a tissue culture to an amount sufficient to protect bovines against disease caused by BRCV or BECV or to identify the molecular structure of BRCV or BECV for preparation of subunit or recombinant products, comprising inoculating BRCV onto a tissue culture which is a cloned human rectal cell (cHRT), preferably HRT-E6 designated as ATCC CRL 12478 and harvesting the grown virus.

This invention also describes a method for propagating a BECV by growing the virus in a tissue culture to an amount sufficient to protect bovines against disease caused by BECV or BRCV or to identify the molecular structure of BECV or BRCV for preparation of subunit or recombinant products, comprising inoculating BECV onto a tissue culture which is a cloned human rectal tumor cell (cHRT), preferably HRT-E6 designated as ATCC CRL 12478 and harvesting the grown virus.

In accordance with the invention, a broadly cross-reactive BRCV or BECV isolate can be used for vaccine preparations described herein. Such cross-reactivity can be demonstrated by *in vitro* cross neutralization studies (see for instance Example 5). Further and more specific details of the of the invention are represented by but not limited by the following examples.

### EXAMPLES

### Example 1

### Cloning of HRT cells to produce cHRT cells

Human adenocarcinoma cells obtained from ATCC (designated CCL 244) were used in producing cHRT cells as follows. The obtained cells were initially planted and maintained in RMPI 1640 with 10% horse serum added, or in DME or MEM with 5-10% fetal bovine serum (FBS). All studies were conducted using DME or MEM with FBS.

Clones of the CCL 244 were obtained by using standard limiting-dilution techniques in 96-well tissue culture plates. This technique involved removing the HRT-18 cells from a vessel in which the cells were growing by addition of Trypsin/EDTA solution containing 0.1% trypsin and 0.04% EDTA, counting the viable cells and making 10-fold dilutions of the cells in DME or MEM plus 10% FBS so that, theoretically, a large portion of the wells of the 96-well plate would have only one cell. The wells were microscopically observed in order to determine which wells truly had a single cell origin. Many wells showed a small cluster of cells, but five wells were chosen because they appeared to have originated from a single cell. After 10 days of growth./maintenance, one well (clone) was trypsinized for additional cloning in a 96-well plate. The same cloning procedure was repeated. In the second cloning, three clones each appeared to develop from a single cell, and the clone in well E6 was chosen as the one to expand and use in subsequent experiments. This clone was designated HRT-E6. The four remaining clones were maintained until there was some assurance that E6 would be acceptable for at least some additional studies, and development into a stable cell line by art-known techniques.

Experiments were conducted to determine whether the parent CCL 244 cells and HRT-E6 cells were susceptible to selected bovine viruses. The viruses included bovine respiratory syncytial virus (BRSV strain 375), bovine virus diarrhea virus (BVDV NADL strain), bovine herpes virus type I (BHV-1 Cooper strain), bovine parainfluenza virus type 3 (PI₃ strain SF-4) and BRCV (strain RA2R7). Fresh monolayers of CCL 244 and HRT-E6 cells were inoculated with the viruses and adsorption was carried out for 30-60 minutes in serum-free medium. This media was removed and discarded and maintenance medium containing 2% FBS was added. The cultures were observed daily for 5-7 days and if no CPE was apparent, culture fluids were either passed directly onto fresh cells (CCL 244 and HRT-E6) or frozen at -70° C and then passed onto the cell monolayers. Three such blind passages were conducted for each virus that did not show CPE. The results for growth of the various viruses on HRT-18G cells have been obtained from the publication by Storz (JAVMA, 1996, 208:9, 1452-1455). The results are tabulated below in Table 1. CCL 244, HRT-E6 and HRT-18G cells were susceptible to BRCV on the first passage. Storz reports that his HRT-18G cells are susceptible for BHV-1. BHV-1 did not show CPE until late in the incubation period of the second passage on the HRT-E6 cells of this invention. This experiment demonstrates that the HRT-E6 cells are phenotypically different from HRT-18G cells and the parent CCL 244 cells.

**Table 1 Virus Susceptibility of CCL 244 and HRT-E6 Cells as Compared with Reported Growth on HRT-18G by Dr. Storz**

| Virus | Growth on CCL 244 | Growth on HRT-E6 | Growth on HRT-18G |
|---|---|---|---|
| BRSV | Negative | Negative | Negative |
| BVDV | Negative | Negative | Negative |
| BHV-1 | Positive | Negative* | Positive |
| P13 | Negative | Negative | Positive |
| BRCV | Positive | Positive | Positive |

### Example 2

### Uniqueness of cHRT cells as Compared with the Parent CCL 244 Cell

To distinguish the uniqueness of the clones of the CCL 244 cells (cHRT) which include HRT-E6, an experiment was conducted to evaluate the cells' BRCV growth characteristics compared with that of several other cells capable of propagating bovine viruses. BRCV isolate RA2R7 available from a calf displaying respiratory disease in South Dakota, was inoculated onto cell lines including Madin Darby Bovine Kidney cells (MDBK), swine testicle cells (ST), feline lung cells (FL), Bayer 9009 cells and HRT-E6 cells. The BRCV isolate and/or its subculture fluids were adsorbed for 60 minutes in serum-free medium. When no CPE was apparent, the cultures were either passaged directly onto fresh cells or frozen and then passaged to the various cell monolayers. Five passages were made on each cell line. None of the cells, except the HRT-E6, showed CPE at any time during the experiment. Therefore, the cHRT cells are unique.

### Example 3

### Uniqueness of cHRT cells as Compared with the Parent CCL 244 cell

To further demonstrate the uniqueness of the cHRT cells including HRT-E6 and HRT-18G, from the parent CCL 244 cells, the following experiment was conducted. Two BRCV isolates, RA2R7 and AZ26649 isolated from a calf in Arizona demonstrating respiratory disease, and one BECV isolate designated 50-3, (obtained from Dr. Johannes Storz) were grown as described in EXAMPLE 1. Each was then titered on either CCL 244 cells, HRT-E6 cells or HRT-18G cells. Titrations were conducted in 96-well plates using fresh monolayers of each of the cells according to art-known procedures. Results are listed in Table 2 as titers expressed as the log₁₀ TCID₅₀/mL.

**Table 2 Titers of BRCV and BECV on cHRT Cells as Compared with their Titers on the Parent CCL 244 Cells**

| Virus Isolate | Titer on CCL 244 Cell | Titer on HRT-18G Cell | Titer on HRT-E6 Cell |
|---|---|---|---|
| RA2R7 | 3.4 | 5.2 | 6.8 |
| AZ26649 | 3.0 | 6.5 | 6.4 |
| 50-3 | 5.6 | 5.4 | 4.7 |

It is apparent that the cHRT cells propagate BRCV isolates to significantly higher titers than does the parent CCL 244 cells. It is noteworthy that all three cells propagated the BECV isolate 50-3 to approximately equivalent titer levels. When propagating BRCV for commercial vaccine purposes, it is important to obtain the highest titers possible. Therefore, the cHRT cells are commercially feasible for propagation of BRCV whereas the CCL 244 cells are not.

### Example 4

### Confirmation of the Difference between BRCV and BECV Isolates

A comparison was made between the various isolates noted in EXAMPLE 3 and other isolates described hereunder to verify that they were BRCV isolates. According to Storz (JAVMA, 1996, 208:9, 1452-1455), BRCV hemagglutinates mouse RBCs and does not hemagglutinate chicken RBCs. In this experiment, isolates RA2R7, AZ26649, LSU051 (BRCV isolate obtained from Dr. Storz), 6J305 (BRCV isolate obtained by ImmTech) and 50-3 were evaluated for hemagglutination activity (HA) against both mouse and chicken RBCs. Virus pools of each of the isolates were tested according to standard hemagglutination techniques using 0.5% mouse or chicken RBCs. The results are expressed as the reciprocal of the highest dilution producing hemagglutination (HA Titer) and are summarized in Table 3. It was clear from the results that the BRCV isolates RA2R7, AZ26649, LSU051 and 6J305 hemagglutinate mouse RBCs, and not chicken RBCs and are, therefore, different from the known BECV isolate, 50-3, which hemagglutinates mouse RBCs to a much higher titer and also shows some hemagglutination of the chicken RBCs.

**Table 3 Hemagglutination Titers of BRCV and BECV Isolates**

| Isolate | HA Titer on Mouse RBCs | HA Titer on Chicken RBCs |
|---|---|---|
| RA2R7 | 256 | <8 |
| AZ26649 | 32 | <8 |
| LSU051 | 64 | <8 |
| 6J305 | 256 | <8 |
| 50-3 | >4096 | 8 |

### Example 5

### Cross-Neutralization of BRCV and BECV isolates

Although Example 4 indicated that BRCV and BECV isolates are distinct in that they show different HA patterns, it is important to note the degree of antigenic relatedness for vaccine development purposes. Therefore, a cross-neutralization experiment was performed. Three BRCV field isolates and one BECV field isolate were evaluated for cross-neutralization. BRCV-CA was isolated from a calf in a California feed lot. BRCV-TX was isolated from a beef calf in a Texas feedlot and BRCV-OK was isolated from an Oklahoma beef calf. BECV 50-3 was again used as the known BECV isolate. Two rabbits were repeatedly injected with purified virus preparations (subunits) in adjuvant prepared from each isolate. After the last injection, the rabbits were bled and sera were collected. The collected sera were evaluated for the ability to neutralize BRCV and BECV isolates in an *in vitro* cell culture assay.

All the virus isolates were purified by ultracentrifugation in a 20-60% linear sucrose gradient. Purified virus, at a concentration of 20-50 µg per dose total protein, was combined with Freund's complete adjuvant (FCA) for the primary rabbit injections and Freund's incomplete adjuvant (FIA) for three subsequent booster injections. Injections were given by the intramuscular route, at 21 day intervals. A final bleed for serum was performed seven days after the last injection.

Cross-neutralization assays were performed by combining isolate specific serum samples with 100-300 TCID₅₀ of each virus isolate. Twofold serial dilutions of serum were made in 96 well plates with two dilution series per serum sample. One hundred to 300 TCID₅₀ of virus was added to all wells and the serum/virus mix was incubated for one hour at 37 ° C. Following incubation, cHRT cells were added to all wells and the plates were incubated at 37° C in a humid, 5% CO₂ incubator for 5-6 days. Table 4 is a summary of the results of the *in vitro* cross-neutralization assay. The resulting immunological activity is represented by serum antibody titers expressed as the reciprocal of the highest dilution of serum which completely neutralized the virus.

The results of this experiment showed that in all cases an antibody response to individual BRCV or BECV isolates could neutralize all of the heterologous isolates to some degree. There were clear cross-neutralization differences between BRCV and BECV. Similarly, there appear to be cross-neutralization differences among BRCV isolates as well. However, the degree of difference demonstrated here is not great enough to classify BRCV and BECV as separate and distinct serotypes.

These data clearly indicate that an antibody response to one BRCV or BECV isolate prepared in accordance with this invention provides enough cross-neutralizing activity to neutralize challenges with heterologous isolates. Specifically, a BRCV based vaccine would elicit an antibody response that would neutralize challenge with BECV, a distinct disease entity as demonstrated above, and other heterologous BRCV isolates. Additionally, it would be expected that a BECV based vaccine would elicit an antibody response that would neutralize challenge with BRCV, a distinct disease entity as also demonstrated above and other heterologous BECV isolates.

**Table 4 Cross-Neutralization Titers of BRCV and BECV isolates.**

| Isolate specific antibody | Rabbit number | Virus Isolates | | | |
|---|---|---|---|---|---|
| | | BRCV-CA 26649 | BRCV-TX 2385-42 | BRCV-OK 0514-50 | BECV 50-3 |
| | | | | | |
| BRCV- | 001 | 512^{a} | 1024 | 512 | 256 |
| CA | 002 | 4096 | 1024 | 512 | 512 |
| 26649/ | Mean | 2304 | 1024 | 512 | 384 |
| Ab | | | | | |
| Folder Difference^{b} | | -- | 2.3 | 4.5 | 5.3 |
| BRCV- | 003 | 1024 | 2048 | 1024 | 256 |
| TX | 004 | 256 | 8192 | 1024 | 1024 |
| 2385-42/ | Mean | 640 | 5120 | 1024 | 640 |
| Ab | | | | | |
| Fold Difference | | 7.7 | -- | 5.0 | 7.7 |
| BRCV- | 005 | 1024 | 4096 | 2048 | 1024 |
| OK | 006 | 1024 | 2048 | 1024 | 512 |
| 0514-50/ | Mean | 1024 | 3072 | 1536 | 768 |
| Ab | | | | | |
| Fold Difference | | 1.5 | 2.0 | 00 | 2.0 |
| BECV | 007 | 1024 | 4096 | 512 | 2048 |
| 50-3/ Ab | 008 | 1024 | 4096 | 256 | 1024 |
| | Mean | 1024 | 4096 | 384 | 14536 |
| Fold Difference | | 1.4 | 2.7 | 4.0 | -- |

| | | | | | |
|---|---|---|---|---|---|
| a = Reciprocal of the last antibody dilution to completely neutralize the virus isolate. | | | | | |
| b = Numerical transformation of the mean homologous antibody/virus titer over each mean heterologous antibody/virus titer. | | | | | |

### Example 6

### Inactivated Bovine Respiratory Coronavirus Vaccine (BRCV)

In the practice of the invention, an antigenic mass of BRCV can be formulated into vaccine which contains an immunogenically effective amount of virus or antigens therefrom. The following is a description of an economically feasible process by which an antigenic mass of BRCV was prepared, inactivated and formulated into an inactivated vaccine form. The vaccine of the invention was administered intramuscularly. However, it could also be administered by a subcutaneous route, or alternatively, by intranasal, intradermal or oral routes. Between one and five milliliters of vaccine may be administered.

BRCV isolate AZ26649 was propagated in cHRT cells described previously (HRT-18G). These cells were most effectively cultured and maintained in growth media consisting of DME supplemented with 5% fetal bovine or donor horse serum and a suitable buffer system such as 1.3g /L sodium bicarbonate. Cell passages were made with cell counts sufficient to attain confluency in 24-48 hours.

Confluent monolayers of cHRT cells were inoculated with BRCV in the following manner. Virus stock was diluted in serum-free DME to achieve a multiplicity of infection (MOI) of one infectious virus particle per 10 to 100 (0.1 to 0.01) cHRT cells. Virus infection of cHRT cells was accomplished by removal of spent growth media, replacement with the dilute virus inoculum at volumes sufficient to cover the monolayer completely and adsorption for 1 hour at 37° C. Following adsorption, a maintenance media consisting of DME supplemented with 2% Fetal Bovine Serum (FBS) and a suitable buffer system such as 1.3g /L sodium bicarbonate was added. Virus infected tissue cultures were incubated at 37° C in a humid, 5.0% CO₂ atmosphere until complete CPE was observed. The CPE observed consisted of rapid cell aggregation and fusion followed by detachment of polykaryons from the substrate resulting in large infected cell aggregates suspended in the maintenance media. Typically, complete CPE was observed and cultures were harvested 2-4 days post-infection. Typically, the TCID₅₀ titer of BRCV obtained ranged from 10 ^{5.5} to 10 ^{8.0} TCID₅₀/mL of harvest fluid.

Harvest fluids containing 10^{6.67} TCID₅₀ /mL of BRCV were inactivated with 0.1% vol/vol BPL using standard methods. Briefly, harvest fluids and BPL were combined and mixed for 24 hours at ambient temperature (approximately 25 ° C). A pH range of 6.8-7.2 was maintained during the inactivation period using 3.0 N NaOH. Non-limiting examples of other suitable inactivating agents or methods envisioned include formaldehyde, binary ethyleneimine, heat, and UV light exposure.

Inactivated BRCV was adjuvanted with Carbopol® ( B.F. Goodrich Co.) as follows. A volume of stock adjuvant (1.5% Carbopol®) was combined with a volume of inactivated virus so that a final dose of vaccine contained 20% adjuvant (vol/vol). Adjuvanted fluids were mixed for at least 24 hours at ambient temperature (approximately 25 °C ). Non-limiting examples of other suitable adjuvants envisioned include; aluminum salts such as aluminum hydroxide and aluminum phosphate; other polymers such as POLYGEN^{™}, DEAE Dextran, Dextran sulfate and methacrylates; IMMUGEN^{™}, dimethyldodecylammonium bromide; poxvirus proteins such as Baypamune; Avirdine, Lipid A; oils such as SUPRIMM^{™}, EMULSIGEN^{™}, EMULSIGEN PLUS^{™} animal oils such as squalane or squalene; mineral oils such as Drakeol and Montanides; vegetable oils such as peanut oil; block co-polymers; triterpenoid glycosides such as saponin, QuilA, and QS21; detergents such as Tween-80 and Pluronic; bacterial component adjuvants such as from Corynebacterium, Propionibacterium, and Mycobacterium; interleukins, monokines, and interferons; liposomes; ISCOMs; synthetic glycopeptides such as muramyl dipeptides and derivatives thereof; cholera toxin; or combinations of the above.

The immunogenic effectiveness of an inactivated BRCV vaccine prepared in the above manner was demonstrated in calves. Vaccines were prepared to contain three different antigen masses based on the preinactivation TCID₅₀ titer of the viral harvest fluids. High, medium, and low antigenic mass vaccines were formulated to contain 10^{5.5}, 10^{6.0}, and 10^{6.5} TCID₅₀ per 2.0 mL dose of BRCV respectively. Each vaccine was administered to four calves and an additional four calves served as the non-vaccinated control group. Calves were vaccinated twice, by the intramuscular route, 19 days apart. Calves were bled prior to primary vaccination, at administration of the second dose, and 7 and 14 days after administration of the second dose for assessment of serum-virus neutralizing (SN) antibody response to BRCV. During the course of the experiment, vaccinated and non-vaccinated animals were co-mingled. Table 5 summarizes the SN immunological activity as demonstrated by this example and as measured as serum neutralizing antibody response of calves post vaccination with the various antigenic masses of inactivated vaccines.

As shown in Table 5 , the results of this study demonstrated that two doses of low, medium, or high antigenic mass vaccine elicited mean fold increases of 8.4, 9.1, and 16 respectively, in virus neutralizing anti-BRCV antibodies. During this same period the mean antibody titer of the co-mingled non-vaccinated control group declined; indicating that no natural exposure to BRCV had occurred during the experiment. Since virus neutralizing antibody was measured and it is generally accepted that a four-fold increase in antibody titer is indicative of significant immunological activity, these data indicate that a immunogenically effective immune response could be elicited using an inactivated BRCV vaccine. An antigenic mass as low as 10^{5.5} TCID₅₀/mL has been shown to be immunogenically effective for an inactivated vaccine.

**Table 5 Serum-virus Neutralizing Antibody Response of Calves Vaccinated with Inactivated Bovine Respiratory Coronavirus Vaccines.**

| Group | Calf # | Primary dose (V1) day 0 | Booster dose (V2) day 19 | 7 days Post-V2 day 26 | 14 days Post-V2 day 33 |
|---|---|---|---|---|---|
| Low AGM | 001 | 8 | 32 | 128 | 256 |
| (10^{5.5} | 002 | 32 | 64 | 32 | 32 |
| TCID₅₀) | 003 | 2 | 4 | 16 | 16 |
| | 004 | <2 | 8 | 64 | 64 |
| | Mean | 11 | 27 | 60 | 92 |
| Medium | 005 | 16 | 8 | 128 | 128 |
| AGM (10^{6.0} | 006 | 32 | 32 | 128 | 128 |
| TCID₅₀) | 007 | 4 | 4 | 128 | 128 |
| | 008 | <2 | <2 | 64 | 128 |
| | Mean | 14 | 15 | 112 | 128 |
| High AGM | 009 | 16 | 64 | 256 | 128 |
| (10^{6.5} | 010 | 2 | 4 | 64 | 128 |
| TCID₅₀) | 011 | 16 | 32 | 128 | 256 |
| | 012 | <2 | 8 | 32 | 64 |
| | Mean | 9 | 27 | 120 | 144 |
| Non- | 013 | 4 | 4 | 2 | 8 |
| vaccinated | 014 | 64 | 16 | 16 | 32 |
| Controls | 015 | 32 | 16 | 32 | 32 |
| | 016 | <2 | <2 | <2 | <2 |
| | Mean | 26 | 10 | 13 | 19 |

| | | | | | |
|---|---|---|---|---|---|
| AGM = Antigenic Mass | | | | | |

### Example 7

### Modified Live Bovine Respiratory Coronavirus Vaccine

BRCV isolate AZ26649 was prepared according to the procedure described in EXAMPLE 6, except it was propagated in the cHRT cell identified as HRT-E6. The virus was grown to a titer of 10^{6.7} TCID₅₀/mL and was harvested by shaking the vessels containing the virus and cells and pouring them into a collecting container. The harvested virus fluids were used to prepare the vaccine for this experiment. Although clarification, concentration, inactivation and adjuvanting were not used to prepare this vaccine, all or part of these processes could be used to produce a higher antigenic mass. For this experiment, the antigenic mass administered to each calf was 5 X 10^{6.7} TCID₅₀. It is within the conception of the invention to add adjuvant to a modified live BRCV or BECV vaccine. In order to prepare a successful adjuvanted modified live vaccine, the BRCV or BECV may have to be further modified (e.g. made less virulent). Preparation of a more modified version of BRCV or BECV would be achieved by passaging the AZ26649 or any other BRCV or BECV through high susceptibility cell such as cHRT cells until it no longer produces any disease signs when administered intranasally to seronegative calves. Another method would be to administer the adjuvanted modified live vaccine by an unnatural route such as intramuscularly, intradermally, subcutaneously or intraperitoneally rather than intranasally. Alternatively, the virus could be treated with mutagenizing agents such as nitrosoguanidine and less virulent mutants could be selected. Mutants would be selected for their inability to produce disease signs when intranasally administered to seronegative calves as well as their continued capability to produce an immunogenically effective vaccine. Such methods of further modifying the isolate will improve the safety of the vaccine.

Three calves (#61, #71 and #74) were approximately eight months of age at primary inoculation (first vaccination). The calves were seronegative for BRCV and received 5.0 mL of the previousiy-described vaccine which was administered intranasally. The vaccinated calves were challenged with 1 X 10^{5.8} TCID₅₀ of BRCV AZ 26649 grown as previously described and incorporated in a volume of 10 mL, twenty-four days after their single primary vaccination. Three non-vaccinated seronegative control calves were challenged with the same amount of BRCV AZ 26649 at the same time. All calves were evaluated daily for twelve days for clinical signs of BRCV disease. Clinical evaluations included measurement of rectal temperatures, observation of respiratory signs (dyspnea, nasal discharge and cough), and observation of gastrointestinal signs (feces, appetite). The scoring system is shown below:

| Nasal Discharge | Fecal Consistency | Dyspnea, Cough Appetite |
|---|---|---|
| 0 = Normal | 0 = Normal | 0 = Normal |
| 1 = Serous | 1 = Pasty | 1 = Mild |
| 2 = Mucous | 2 = Semi-liquid | 2 = Moderate |
| 3 = Mucopurulent | 3 = Liquid | 3 = Severe |
| 4 = Epistaxis | 4 = Bloody | |

The column marked Clinical Scores in Table 6 was a compilation of the total of the above-identified Nasal Discharge and Dyspnea, Cough, Appetite scores (Respiratory Disease) divided by the number of animals in each group (average). The Enteric Scores in Table 6 represent the average Fecal Consistency scores as identified above. Daily nasal swabs were collected in MEM containing 5% Gentamicin and stored at -70°C after being filtered through 0.45 µm syringe filters. The nasal swabs were later tested for presence of virus by known methods of inoculation of the cHRT cells in a tissue culture system. Table 6 below shows the average rectal temperatures, average total clinical scores and virus isolation results for the three vaccinated calves (vaccinates) as compared with the three non-vaccinated calves (controls) during the twelve days post challenge.

Results indicate that vaccinated calves demonstrated a significant reduction in Respiratory Disease (59%) as well as a significant reduction in Enteric Disease (59%). One vaccinated calf displayed an Enteric Score of 1.0 on day 0 which lasted through day 11. If this calfs result is removed from the study, the BRCV vaccine would show a 100% reduction in Enteric Disease. A 50% reduction was observed in days of virus shedding of vaccinates as compared with controls. No significant temperature responses were noted. These data indicate that a BRCV vaccine can protect against both the respiratory as well as the enteric form of the coronavirus disease.

**Table 6 Results of the BRCV Vaccination/Challenge Study**

| Treatment Group | Day Post Chall. | Temperature | Clinical Score | Enteric Score | Positive Isolation |
|---|---|---|---|---|---|
| Vaccinate | 0 | 102.0 | 0.33 | 0.33 | 0/3 |
| Control | 0 | 102.0 | 0.00 | 0 | 0/3 |
| Vaccinate | 1 | 102.1 | 0.67 | 0.33 | 1/3 |
| Control | 1 | 102.1 | 1.00 | 0.33 | 1/3 |
| Vaccinate | 2 | 102.0 | 1.00 | 0.33 | 1/3 |
| Control | 2 | 102.6 | 1.00 | 0.67 | 2/3 |
| Vaccinate | 3 | 102.2 | 1.00 | 1.00 | 2/3 |
| Control | 3 | 102.8 | 2.30 | 1.67 | 2/3 |
| Vaccinate | 4 | 102.0 | 2.00 | 0.33 | 2/3 |
| Control | 4 | 102.8 | 3.33 | 1.67 | 3/3 |
| Vaccinate | 5 | 102.0 | 1.00 | 0.33 | 1/3 |
| Control | 5 | 102.4 | 3.00 | 1.33 | 3/3 |
| Vaccinate | 6 | 102.2 | 1.00 | 0.33 | 2/3 |
| Control | 6 | 102.6 | 1.33 | 1.33 | 3/3 |
| Vaccinate | 7 | 101.0 | 1.00 | 0.33 | 2/3 |
| Control | 7 | 102.4 | 4.00 | 0.67 | 3/3 |
| Vaccinate | 8 | 102.0 | 2.33 | 0.33 | 1/3 |
| Control | 8 | 102.3 | 2.33 | 0.67 | 3/3 |
| Vaccinate | 9 | 101.8 | 0.67 | 0.33 | 1/3 |
| Control | 9 | 101.9 | 2.33 | 0.67 | 3/3 |
| Vaccinate | 10 | 101.8 | 0.67 | 0.33 | 1/3 |
| Control | 10 | 102.0 | 1.33 | 0.67 | 2/3 |
| Vaccinate | 11 | 101.9 | 0.33 | 0.33 | 0/3 |
| Control | 11 | 101.8 | 1.67 | 0.33 | 2/3 |
| Vaccinate | 12 | 101.7 | 0.00 | 0 | 0/3 |
| Control | 12 | 101.8 | 1.00 | 0.33 | 1/3 |

To further demonstrate the immunological activity of the BRCV vaccine of this example, serum was collected from each vaccinated calf on days 0 and 24 for evaluation of serum neutralizing antibodies to BRCV as a result of vaccination and on days 0 and 17 post challenge to demonstrate that the calves were truly exposed to the virus. Table 7 shows the serum neutralization titers of all calves expressed as the reciprocal of the last dilution to produce neutralization. A single vaccination with the modified live BRCV vaccine produced significant titers in all vaccinated calves. Control calves remained seronegative indicating that there was no exposure to BRCV prior to challenge. After challenge the vaccinated calves demonstrated an anamnestic response.

**Table 7 Serum Neutralization Titer of Calves Post-Vaccination, Pre-Challenge and Post-Challenge**

| Calf No./ Status | Titer on Day 0 (Day of Vacc) | Titer on Day 24 (Day of Chall.) | Titer on Day 17 Post Challenge |
|---|---|---|---|
| 61/Vacc | <2 | 64 | 512 |
| 71/Vacc | <2 | 16 | 64 |
| 74/Vacc | <2 | 128 | 256 |
| 54/Cont | <2 | <2 | 64 |
| 62/Cont | <2 | <2 | 32 |
| 70/Cont | <2 | <2 | 8 |

| | | | |
|---|---|---|---|
| Vacc = Vaccinate | | | |
| Cont = Control | | | |

### Example 8

### Preparation of a Subunit Vaccine

Cloned HRT cells are infected with BRCV according to the method described in Example 6. The infected cells are harvested between 12 and 72 hours post-infection when CPE is >80%. Infected cells are prepared for extraction by harvesting the infected tissue culture fluids, such harvesting involving pouring the infected tissue culture into a collection vessel which can then be centrifuged at low speeds (approximately 1000 x g). The infected tissue culture can be centrifuged in batches using a normal centrifuge or in a continuous flow centrifuge. After centrifugation, the cell pellet is extracted by addition of a detergent in a buffer system. In this example the buffer-detergent is phosphate buffered saline (PBS) plus 1.0% Nonidet P-40. This buffer-detergent is used to resuspend the cell pellet and extraction is carried out by mixing the suspended infected cell pellet at 4°C until the cell pellet is uniformly solubilized or approximately 30 to 120 minutes. Following this extraction, any insoluble material is removed by low speed centrifugation (batch or continuous flow) and may be re-extracted as described above. Solubilized extracts are then combined and purified by column chromatography (size exclusion, lectin or other affinity, anion/ cation exchange, and/or reverse phase) before adjuvanting or extracts may be adjuvanted directly. The antigenic mass is measured by art-known methods such as enzyme linked immunoassay (ELISA) prior to adjuvanting. If the antigenic mass is high enough the extract or purified extract may be diluted in PBS. If the antigenic mass is to low to be immunogenically effective, the extract or purified extract may be concentrated using ultrafiltration or other such concentration methods. Adjuvanting can be conducted using any of the adjuvants described above.

Given the above disclosures, it is expected that numerous variations in our BRCV and BECV vaccine preparation and use as well as preparation of the cHRT cells, will occur to those skilled in the art. Thus, it is intended that the above-described examples should be construed as illustrative only and that the scope of the invention disclosed herein should be limited only by the following claims.

## Claims

1. An improved cloned human rectal tumor cell, HRT-E6, designated ATCC CRL 12478.

2. A method of propagating BRCV, bovine respiratory coronavirus, or BECV, bovine enteric coronavirus, to a high antigenic mass, comprising the steps of:
a. propagating the cell of claim 1 on a vessel surface or in suspension in the presence of a tissue culture medium to produce an acceptable viable cell count;
b. inoculating the propagated the cell of claim 1 with BRCV or BECV to produce an infected cell culture;
c. incubating said infected cell culture at 30 to 38"C to produce an acceptable CPE;
d, harvesting the resulting infected cell culture by transfer into a collecting container;
e. optionally disrupting said infected cell culture; and
f. optionally concentrating said infected cell culture to a high antigenic mass.

3. A method for propagating a bovine respiratory coronavirus (BRCV) by growing the virus in a tissue culture to an amount sufficient to protect bovines against bovine respiratory disease, or to identify the molecular structure of BRCV or BECV for preparation of subunit or recombinant products, comprising the steps of inoculating BRCV onto a tissue culture which is the cell of claim 1 and harvesting the grown virus.

4. A method for propagating a bovine enteric coronavirus by growing the virus in a tissue culture to an amount sufficient to protect bovines against bovine respiratory coronavirus disease and bovine enteric coronavirus disease or to identify the molecular structure of BECV or BRCV for preparation of subunit or recombinant products, comprising inoculating BECV onto a tissue culture which is the cell of claim 1.

## Patentansprüche

1. Verbesserte klonierte, menschliche Rektaltumorzelle, HRT-E6, bezeichnet als ATCC CRL 12478.

2. Verfahren zur Vermehrung von BRCV (bovinem respiratorischen Coronavirus) oder BECV (bovinem enteralem Coronavirus) zu einer hochantigenen Masse, folgende Schritte umfassend:
a. Vermehren der Zelle nach Anspruch 1 an einer Gefäßoberfläche oder in Suspension in Gegenwart eines Gewebekulturmediums, um eine annehmbare Lebendzellzahl zu produzieren;
b. Inokulieren der vermehrten Zelle nach Anspruch 1 mit BRCV oder BECV, um eine infizierte Zellkultur zu bilden;
c. Inkubieren der infizierten Zellkultur bei 30 bis 38 °C, um einen annehmbaren CPE zu erzielen;
d. Ernten der resultierenden infizierten Zellkultur mittels Transfer in einen Sammelbehälter;
e. gegebenenfalls Aufbrechen der infizierten Zellkultur; und
f. gegebenenfalls Einengen der infizierten Zellkultur zu einer hochantigenen Masse.

3. Verfahren zur Vermehrung eines bovinen respiratorischen Coronavirus (BRCV) durch Züchten des Virus in einer Gewebekultur bis zu einer Menge, die ausreicht, um Rinder gegen bovine Atemwegserkrankungen zu schützen oder um die Molekülstruktur von BRCV oder BECV zur Herstellung von Untereinheits- oder Rekombinationsprodukten zu identifizieren, umfassend die Schritte des Inokulierens von BRCV auf eine Gewebekultur, die eine Zelle nach Anspruch 1 ist, und des Erntens des gezüchteten Virus.

4. Verfahren zur Vermehrung eines bovinen enteralen Coronavirus durch Züchten des Virus in einer Gewebekultur bis zu einer Menge, die ausreicht, um Rinder gegen bovine respiratorische Coronaviruserkrankung und bovine enterale Coronaviruserkrankung zu schützen oder um die Molekülstruktur von BECV oder BRCV zur Herstellung von Untereinheits- oder Rekombinationsprodukten zu identifizieren, umfassend das Inokulieren von BECV auf eine Gewebekultur, die eine Zelle nach Anspruch 1 ist.

## Revendications

1. Cellule tumorale rectale humaine clonée améliorée, HRT-E6, désignée par ATCC CRL 12478.

2. Procédé pour multiplier un coronavirus respiratoire bovin (BRCV) ou un coronavirus entérique bovin (BECV) jusqu'à une masse antigénique importante, comprenant les étapes consistant à :
a. multiplier la cellule de la revendication 1 sur la surface d'un récipient ou en suspension en présence d'un milieu pour culture de tissus afin de produire une numération cellulaire viable acceptable ;
b. inoculer à la cellule multipliée de la revendication 1 le BCRV ou BECV pour produire une culture cellulaire infectée ;
c. mettre en incubation ladite culture cellulaire infectée à une température de 30 à 38°C pour produire un effet cytopathogène (ECP) acceptable ;
d. recueillir la culture cellulaire infectée résultante par transfert dans un récipient collecteur ;
e. facultativement, dissocier ladite culture cellulaire infectée ; et
f. facultativement, concentrer ladite culture cellulaire infectée jusqu'à une masse antigénique importante.

3. Procédé pour la multiplication d'un coronavirus respiratoire bovin (BRCV) par croissance du virus dans une culture de tissu jusqu'à une quantité suffisante pour protéger les bovins contre la maladie respiratoire bovine, ou pour identifier la structure moléculaire du BRCV ou BECV à des fins de préparation de produits consistant en sous-unités ou de produits recombinants, comprenant les étapes d'inoculation du BRCV sur une culture de tissu qui est la culture des cellules de la revendication 1, et à recueillir le virus qui s'est multiplié.

4. Procédé pour multiplier un coronavirus entérique bovin par croissance du virus dans une culture de tissu jusqu'à une quantité suffisante pour protéger les bovins contre la maladie provoquée par le coronavirus respiratoire bovin et la maladie provoquée par le coronavirus entérique bovin, ou pour identifier la structure moléculaire du BECV ou BRCV à des fins de préparation de produits à base de sous-unités ou de produits recombinants, comprenant l'inoculation du BECV sur une culture de tissu qui est la culture des cellules de la revendication 1.
